(19) Europäisches Patentamt
European Patent Office
Office européen des brevets

(11) Publication number: **0 187 674 A2**

(12) # EUROPEAN PATENT APPLICATION

(21) Application number: 86100242.6

(22) Date of filing: 09.01.86

(51) Int. Cl.[4]: C 07 C 29/64
C 07 C 33/46, C 07 C 67/343
C 07 C 69/675, C 07 C 69/757
C 07 D 213/30, C 07 C 79/22
C 07 D 333/16, C 07 C 43/23
C 07 C 41/30, C 07 C 17/00

(30) Priority: 11.01.85 IT 1908085

(43) Date of publication of application: 16.07.86 Bulletin 86/29

(84) Designated Contracting States: BE CH DE FR GB LI NL

(71) Applicant: ISTITUTO GUIDO DONEGANI S.p.A.
Via Caduti del Lavoro
I-28100 Novara(IT)

(72) Inventor: Massardo, Pietro, Dr.
5, via Fra'Bartolomeo
I-20123 Milan(IT)

(72) Inventor: Bettarini, Franco, Dr.
4/B, via Cadore
I-28100 Novara(IT)

(72) Inventor: Piccardi, Paolo
8, via E. De Marchi
I-20125 Milan(IT)

(72) Inventor: Rama, Franco, Dr,
1, via Rodi
I-21052 Busto Arsizio Varese(IT)

(74) Representative: Weinhold, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **Process for the preparation of polyhalogenated carbinols.**

(57) There is disclosed a process for preparing polyhalogented carbinols having formula:

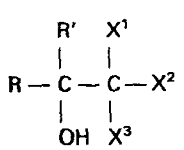

(I)

by reacting a compound having the formula:

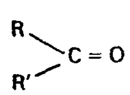

(II)

with a system consisting of a compound having the formula:

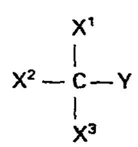

(III)

and of a divalent metal or of a metal salt, in (an) aprotic dipolar solvent(s),

wherein R = H, an alkyl, cycloalkyl, alkenyl, cycloalkenyl, alkadienyl, phenyl, naphthyl, anthracyl group, a heterocyclic radical, each optionally substituted;

R' = H, a haloalkyl, -CN, -COOR" group, R" being H or an alkyl group,

$X^1$ = Cl, Br; $X^2$ = Cl, Br;

$X^3$ = Cl, Br, CF; $CCl_3$; Y = Br or Cl when $X^1$, $X^2$ and $X^3$ are different from Br.

EP 0 187 674 A2

Croydon Printing Company Ltd.

The present invention relates to a process for preparing polyhalogenated carbinols by reacting a carbonylic compound with a system consisting of a polyhalogenated hydrocarbon and of a divalent metal or of a metal salt in (an) aprotic dipolar solvent(s).

The polyhalogenated carbinols are important intermediate products in the fine chemistry and in particular in the field of pesticides for instance for the preparation of benzoylureas and pyrethroides having an insecticidal activity.

In literature the preparation of a few polyhalogenated carbinols was disclosed, in particular the carbinols containing $-C\ Cl_3$ or $-C\ Br_3$ groups, using synthesis methods different from the ones of the present invention, such as for instance the ones described in: Journal of American Chemical Society, 1948 pages 1189, and 1950 pages 5012-14, Bulletin de la Société Chimie de France, 1967, pages 1520-32, British patent application 2,076,804 and Japanese patent application 77/73.842.

Now a simple and economic method has been found, that allows to obtain polyhalogenated carbinols with good yields. Therefore the subject of the present invention is a process for preparing polyhalogenated carbinols having the general formula:

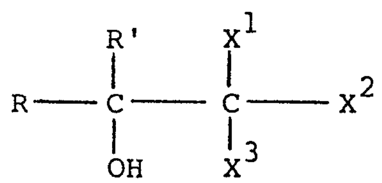

(I)

characterized in that a carbonylic compound having the formula:

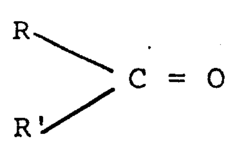

(II)

is reacted with a system consisting of a polyhalogenated hydrocarbon having the formula:

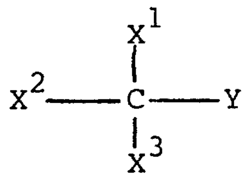

(III)

and of a divalent metal or of a metal salt, in an aprotic dipolar solvent, at temperatures ranging between -20°C and the boiling temperature of the solvent, in said formulas

- R represents: a H atom, an alkyl, cycloalkyl, alkenyl, cycloalkenyl, alkadienyl group, said groups may be substituted, preferably at the first two carbon atoms of the aliphatic chain, by one or more groups selected from a halogen atom, aryl groups, -CN, $-NO_2$, -COOR", wherein R" is H or a $C_1$-$C_4$ alkyl group, said R may further represent a phenyl, naphthyl, anthracyl group, a heterocyclic radical, each optionally substituted by one or more groups selected from halogen atoms, alkyl, alkenyl, alkoxyl, $-NO_2$, -CN, haloalkyl, haloalkenyl, haloalkoxy, thioalkyl, alkylamino, dialkylamino, -COOR", -COR" groups;
- R' represents a H atom, a haloalkyl, -CN, -COOR" group;
- $X^1$ represents Cl, Br;
- $X^2$ represents F, Cl, Br;
- $X^3$ represents Cl, Br, $CF_3$, $CCl_3$;
- Y represents Br or Cl when $X^1$, $X^2$ and $X^3$ are different from Br.

According to special embodiments of the present invention the alkyl, cycloalkyl, alkenyl, cycloalkenyl, alkadienyl (if they are in unsubstituted form) alkoxyl, haloalkyl, haloalkenyl, haloalkoxy, thioalkyl, alkylamino and dialkylamino groups are based on corresponding lower groups, i.e. groups containing 1-6 carbon atoms. The heterocyclic radical may contain e.g. an oxygen, nitrogen and/or sulfur atom.

As metal use may be made for instance of: zinc, copper, iron, magnesium, and as metal salt for instance use may be made of stannous chloride.

As solvent use may be made of: dimethylformamide, dimethylsulfoxide, hexamethylphosphorotriamide, N-methylpyrrolidone, sulfolane, N,N-dimethyl-imidazolindinone.

The reactions, which take place successively during the process can be represented schematically by the following equations:

1)

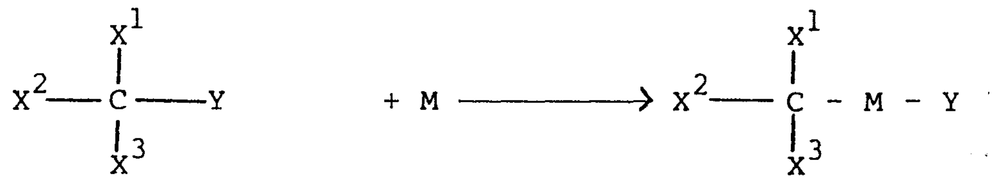


(III) (III M)

2)

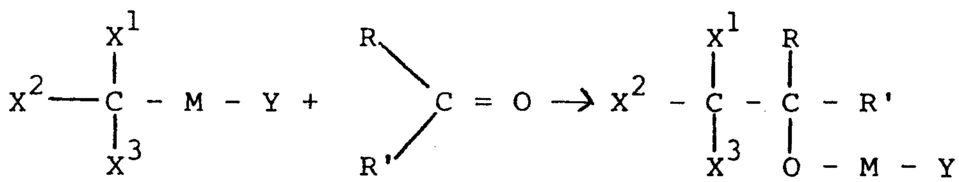

(III M) II (I M)

3)

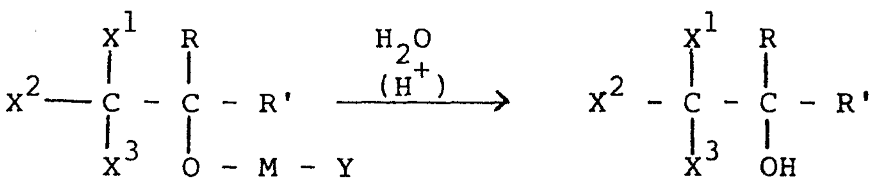


(I M) (I)

wherein M represents the metal.

The operative conditions for the addition of the reactants in the process, according to the present invention, can vary according to the type of compounds that have been utilized and, anyhow, said operative conditions are not a critical factor. Operative conditions concerning the addition may be the following: adding the metal in only one solution or in subsequent shares to a mixture, consisting of compound III, of carbonylic compound II and of the reaction solvent, alternatively compound III, optionally dissolved in the reaction solvent, can be added, to a suspension of the metal in the reaction solvent, in the presence of the carbonylic compound II.

Said operative conditions have to be recommended when the organometallic compound having the formula (III M), which forms during the reaction, turns out not to be very stable and therefore it tends to give rise to undesirable by-reactions.

If the organometallic compound (III M) is sufficiently stable, such as for instance 1,1-dichloro-2,2,2-trifluoroethyl zinc chloride, it is possible to work according to another operative condition consisting in preparing separately the compound (III M), by heating the metal in a suitable solvent with a slight excess of the compound III till dissolution and then in mixing compound (III M) with the carbonylic compound II dissolved in the reaction solvent.

The process can be carried out using the reactants in ratios substantially stoichiometric; it would be better, however, according to the characteristics of the concerned reactants, to use an excess with respect to the stoichiometric one, i.e. of the metal with respect to the carbonylic compound II as well as of the compound III with respect to the metal.

The compounds of general formula I, wherein $X^3$ represents $CF_3$ and the compounds having the general formulas

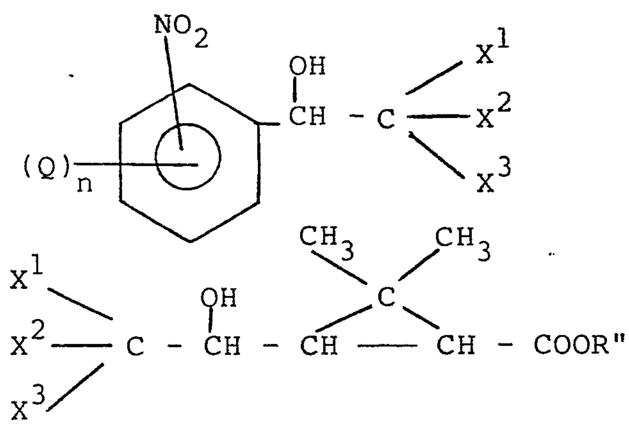

(IV) or

(V)

wherein $X^1$, $X^2$, $X^3$ and R" have the meaning as defined in formula (I); Q represents H, a halogen atom, a $C_1$-$C_3$ alkyl group optionally substituted by 1-3 halogen atoms, $-OCH_3$,

$-SCH_3$, $-OCF_3$; n is an integer comprising 1 to 4, are new compounds and form a further subject of the present invention.

Furthermore it has been found that the carbinols having general formula (I) can be reduced to alkenes having the general formula:

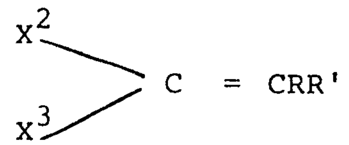

(VI)

using the known methods such as for instance:

a) direct treatment of carbinol (I) with zinc in acetic acid

b) conversion of carbinol (I) to acetate or to dihalophosphite and subsequent treatment with zinc in dimethylformamide

c) substitution of the hydroxyl radical of carbinol (I) with a halogen atom and subsequent dehalogenation by means of zinc.

In the aforesaid conversions it is generally possible to use carbinol (I) in the same reaction medium in which it has been prepared according to the present invention, namely without isolating it, with considerable economic advantages, as well as with advantages concerning the simplicity of carrying out the process for obtaining the corresponding alkenes.

Alkenes of formula (IV) are, in turn, important intermediate compounds for the preparation of pesticides. In particular carbinols of formula (IV) can be reduced, according to aforesaid methods, to alkenes having the formula:

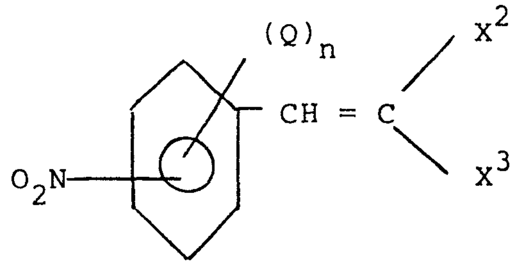

(VII)

which, after reduction to the corresponding anilines and reaction with suitable isocyanates, provide insecticide benzoylureas.

The carbinols of formula (V) can be reduced, according to the aforesaid methods, to alkenes of the formula:

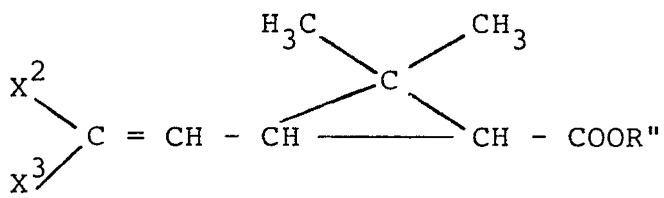 (VIII)

which are successively converted to the respective acid chlorides and then they are esterified to give rise to pyrethroides, insecticides having a very high activity and a large action spectrum.

The following examples will illustrate the invention.

EXAMPLE 1

Preparation of 1-phenyl-2,2,2-trichloro-ethanol.

A solution containing 3.18 g of benzaldehyde (30 millimoles), 6.16 g of carbon tetrachloride (40 millimoles) in 30 ml of DMF, dehydrated on molecular sieves, was loaded into a small 3 neck flask having a capacity of 100 ml provided with a reflux condenser, thermometer, magnetic stirrer and kept under a nitrogen atmosphere.
720 mg (30 milligram atoms) of magnesium in the form of shavings were added in doses; in the beginning the mixture was heated to about 40°C, then after the reaction had started, the metal addition and the thermal regulation were checked in order to keep the inner temperature at about 50°C.
When magnesium was almost completely dissolved, it was heated at 60°C for 4 h and then kept at room temperature overnight.
The reaction mixture was diluted with 250 ml of water, acidified with hydrochloric acid at 10 % and extracted with 3 shares of ethyl ether each of them corresponding

to a volume of 80 ml; the organic extract was washed with 50 ml of a saturated solution of sodium chloride, dehydrated with sodium sulphate, concentrated. The residue was analysed by a chromatographic method on a silica gel column, eluting with hexane/ethyl acetate in a 95:5 ratio.
3.2 g of the reaction product were obtained.
$^1$H-NMR ($CDCl_3$,TMS), $\delta$ (ppm): 3.35 (1H, S); 5.13 (1H, S) 7.1-7.7 (5H, m)
IR ($cm^{-1}$) : 3440 (-OH)

EXAMPLE 2

Preparation of 1-phenyl-2,2,2-tribromo-ethanol.

2.12 g of benzoic aldehyde (20 millimoles), 25 ml of DMF, 6.63 g of carbon tetrabromide (20 millimoles) were loaded into a small 3 neck flask having a capacity of 100 ml, provided with a reflux condenser, thermometer, magnetic stirrer and kept under a nitrogen atmosphere.
1.3 g of zinc (20 milligram atoms) were added in small doses in order to keep the temperature at about 45°C; after having finished the addition, it was heated at 50°C for 1 h. After cooling the mixture was poured into 200 ml of water, acidified with HCl at 10 %, and extracted with 3 shares of ethyl ether, each of them corresponding to a volume of 75 ml, the organic extract was washed with a solution of sodium chloride, dehydrated and concentrated. The product was purified by a chromatographic method on silica gel, eluting with hexane/ethyl acetate having a 95:5 ratio; 3.3 g of a solid were obtained having a m.p. of 73°C.
$^1$H-NMR ($CDCl_3$, TMS), $\delta$ (ppm) : 3.80 (1H, S); 5.18 (1H, S) 7.1-7.8 (5H, m)

EXAMPLE 3

Preparation of ethyl 2-hydroxy-2-tribromomethyl-propionate.

3.31 g of carbon tetrabromide (10 millimoles), 1.16 g of ethyl pyruvate (10 millimoles) and 10 ml of DMF were mixed under a nitrogen atmosphere in a small flask having a

capacity of 100 ml.
650 mg (10 milligram atoms) of zinc in powder form were added, in subsequent shares, under stirring; the temperature of the reaction mixture was kept at about 45°C.

The heating was continued at such temperature for 2 h, then it was diluted with 120 ml of water and 30 ml of a saturated solution of ammonium chloride, extracted with 3 shares of ethyl ether, each of them having a volume of 50 ml, dehydrated with sodium sulphate and concentrated in a rotary evaporator. The raw product of reaction was purified by column chromatography, eluting with hexane/ ethyl acetate having a 95:5 ratio; 1.6 g of product were obtained.
$^1$H-NMR ($CDCl_3$, TMS), $\delta$(ppm): 1.35 (3H, t); 1.85 (3H, S) 4.35 (2H, q); 4.45 (1H, S)
IR ($cm^{-1}$): 3460 (-OH); 1740 (-COOEt).

EXAMPLE 4

Preparation of ethyl 2-(2,2-dichloro-1-hydroxy-3,3,3-trifluoro-propyl)-3,3-dimethyl-cyclopropane-carboxylate.

9.8 g of Zn in powder (0.15 gram atoms) and 37.5 g of 1,1,1-trichloro -2,2,2-trifluoro-ethane (0.2 moles) in 125 ml of anhydrous THF were reflux heated under a nitrogen atmosphere, till dissolution of the metal; solvent and freon in excess were removed by distillation at reduced pressure.
The residue was dissolved in 50 ml of DMF and 17 g (0.1 moles) of ethyl 3,3-dimethyl-2-formyl-cyclopropanecarboxylate (cis-trans mixture) dissolved in 50 ml of DMF were added to said residue.
Always under a nitrogen atmosphere and under stirring, it was heated at 45°C for 5 hours.
After cooling it was diluted with 500 ml of acidulated water and extracted with 3 shares of ethyl ether, each of them having a volume of 150 ml; the extract was washed with a solution of sodium chloride (100 ml), then dehydrated and concentrated.

0187674

After separation by column chromatography (eluent:hexane/ ethyl acetate 95:5) 18.5 g of carbinol (cis-trans mixture 45:55) were obtained.
$^{1}H$-NMR ($CDCl_3$, TMS), $\delta$(ppm): 1.1-1.4 (9H, m) 1.55-2.1 (2H, m); 2.7 (1H, S); 3.75 (1H, d); 4.08 (2H, q)

EXAMPLE 5

Preparation of 2,2-dibromo-3,3-trifluoro-1-(2-chlorophenyl)propanol.

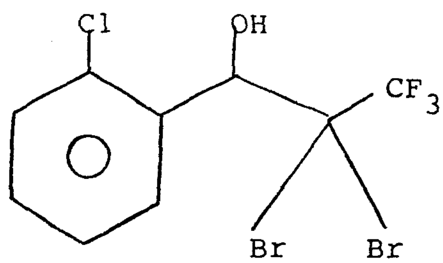

83.5 g of $CF_3CBr_3$ in anhydrous DMF and 28 g of 2-chlorobenzaldehyde were reacted under a nitrogen atmosphere. The mixture was brought to 5°C and zinc in powder (16.0 g) was added to it slowly. Then the mixture was let reach the room temperature and kept under stirring for 6 hours. The mixture was poured into water and extracted three times with ethyl ether.
The ethereal extract was shaken with an aqueous solution of sodium bisulfite in order to eliminate the non-reacted aldehyde, and then was dehydrated with anhydrous sodium sulphate. After filtration, the residue was concentrated under vacuum and purified by chromatography on silica gel, eluting with hexane/ether 95:5.
30 g of product were thus obtained.
$^{1}H$-NMR ($\delta$, THS = 0); 8.0-7.4 (m,4H, Arom.); 6.9 (d, 1H, CH); 3.3 (s broad, 1H, OH)

EXAMPLE 6

Preparation of 2.2-dichloro-3,3,3-trifluoro-1-phenyl-propanol.

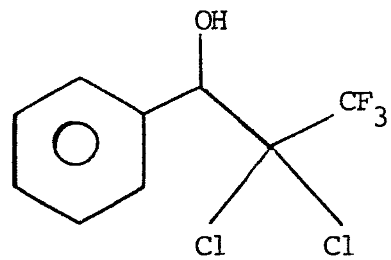

3 g of benzaldehyde and 5.3 g of $CF_3$-$CCl_3$ were reacted in 30 ml of anhydrous DMSO, under a nitrogen atmosphere. 5.3 g of anhydrous $SnCl_2$ were added at room temperature and the mixture was kept at room temperature under stirring for 10 hours. Then the whole was poured into water and extracted with ether.
The ethereal solution was shaken with a solution of sodium bisulfite in order to eliminate the non-reacted benzaldehyde and then dehydrated on anhydrous sodium sulphate. The ether was removed under vacuum and 4.4 g of product were obtained.
$^1$H-NMR($CDCl_3$, TMS) $\delta$ (ppm); 2.9 (1 H, S) OH; 5 (1 H,S) CH; 7.2 (5 H, m) aromatic.

EXAMPLE 7

Preparation of 2,2-dichloro-3,3,3-trifluoro-1-(3-pyridyl)-1-propanol.

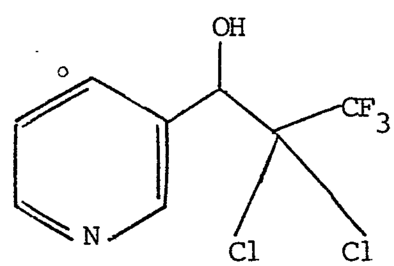

5 g of 3-pyridine-carboxy-aldehyde in 100 ml of anhydrous DMF and 34 g of $CF_3CCl_2$.ZnCl prepared previously were reacted under a nitrogen atmosphere. The mixture was heated at 40° for 4 h, then it was poured into water and extracted with ether several times. The collected organic extract was dehydrated on anhydrous sodium sulphate and then concentrated under vacuum. 9 g of a white solid were obtained having a melting point of 141 - 143°C.

EXAMPLE 8

Preparation of 2,2-dichloro-3,3,3-trifluoro-1-(4-nitrophenyl)-propanol

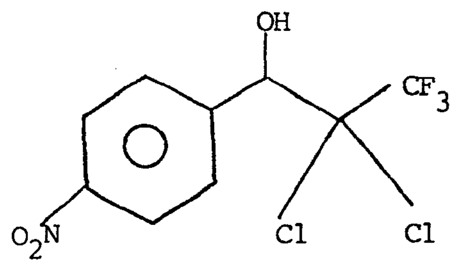

0.066 moles of p-nitrobenzaldehyde and 0.080 moles of complex Zn-$CF_3CCl_3$ prepared previously were reacted in 100 ml of anhydrous DMF under a nitrogen atmosphere. It was heated at 35-40°C for 3 h. The whole was then poured into water, acidified with dilute HCl and extracted 3 times with ethyl ether. The collected organic extract was brought to a neutral pH by means of washings with water and after dehydration on anhydrous sodium sulphate it was concentrated under vacuum.
18 g of a clear solid were thus obtained, having a melting point of 102°C.

EXAMPLE 9

Preparation of 2,2-dichloro-3,3,3-trifluoro-1-(2,6-dichloro-4-nitrophenyl)-1-propanol

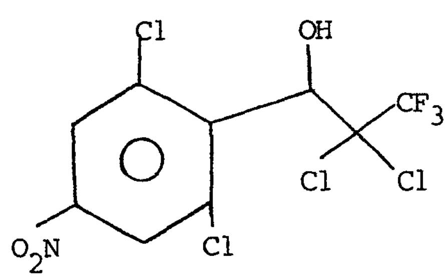

3 g of 2,6-dichloro-4-nitro-benzaldehyde were reacted, in 20 ml of anhydrous DMF under a nitrogen atmosphere, with $CF_3CCl_2ZnCl$ prepared from 5 g of $CF_3CCl_3$ and 2.2 g of Zn in 10 ml of THF at 60°C for 1 hour. The mixture was heated at 50°C for 2 hours and at the end of the reaction it was poured into acidulated water and extracted repeatedly with ethyl ether.

After having removed the ethyl ether under vacuum, 3.5 g of product were obtained.

$^1$H-NMR ($CDCl_3$, TMS) $\delta$ (ppm): 4.3 (1H, d); 6.35 (1H, d); 8.15 (2H, s).

EXAMPLES 10-24

By working under the operative conditions of the preceding examples 15 carbinols were prepared, whose characteristics are reported on Table I.

T A B L E   I

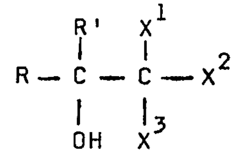

| Ex.No. | R | R' | $X^1$ | $X^2$ | $X^3$ | $^1$H-NMR (δ, TMS=0) | Mass spectrum m.p. |
|---|---|---|---|---|---|---|---|
| 10 | 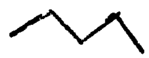 | H | Cl | Cl | $CF_3$ | | m/e 223; 181; 156; 151; 101; 55 (base peak) |
| 11 | 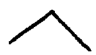 | H | Br | Br | Br | | m/e 279; 243; 73; 55 (base peak) m.p. 54 - 55° |
| 12 | $-C\ Cl_3$ | H | Cl | Cl | $CF_3$ | | m/e 263; 181 (base peak) 151; 147; 111 |
| 13 | 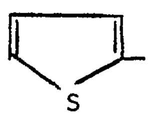 | H | Cl | Cl | $CF_3$ | 7.6-6.8 (m,3H); 5.4 (s broad, 1H); 3.9 (s broad; 1H) | m/e 264 ($H^t$); 247; 212; 113 (base peak) |
| 14 | 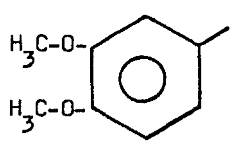 | H | Cl | Cl | $CF_3$ | 7.1-6.7 (m, 3H,Arom) 5.2 (d, 1H, CH); 3.9 (s, 6H, $CH_3$); 3.2 (d, 1H, OH) | m.p. 118-120°C |

T A B L E I (follows)

| Ex.No. | R | R' | $X^1$ | $X^2$ | $X^3$ | $^1$H-NMR ($\delta$, TMS=0) | Mass spectrum m.p. |
|---|---|---|---|---|---|---|---|
| 15 | 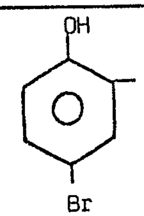 | H | Cl | Cl | $CF_3$ | 10.9 (s, 1H, phenolic OH); 7.5-6.7 (m, 3H Arom); 5.5 (s, 1H, CH); 4.5 (broad, 1H, OH) | |
| 16 | 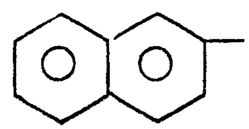 | H | Cl | Cl | $CF_3$ | 7.9-7.4 (m, 7H, Arom); 5.3 (s broad, 1H, CH); 3.7 (s broad 1H, OH) | m.p. 56-58°C |
| 17 | $OHCCH_2$- | H | Cl | Cl | $CF_3$ | | m/e 206; 178; 171; 151; 109; 45 (base peak) |
| 18 | $CH_3$-$CH_2$-$CH_2$-CH=CH- | H | Cl | Cl | $CF_3$ | | m/e 221; 156; 151; 99; 57 (base peak) |
| 19 | 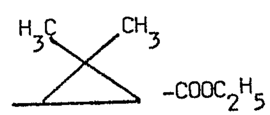 | H | Br | Br | $CF_3$ | 4.1 (q, 2H, $CH_2$); 3.7 (d, 1H, CHOH); 2.8 (s broad, 1H, OH); 2.1-1.6 (m, 2H); 1.4-1.2 (m, 9H, $CH_3$) | |

T A B L E   I   (follows)

| Ex.No. | R | R' | $X^1$ | $X^2$ | $X^3$ | $^1$H-NMR ($\delta$, TMS=0) | Mass spectrum m.p. |
|---|---|---|---|---|---|---|---|
| 20 | 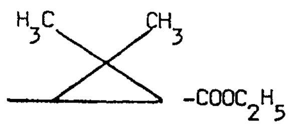 | H | Br | F | $CF_3$ | | m/e 335; 305; 225; 179; 141 |
| 21 | 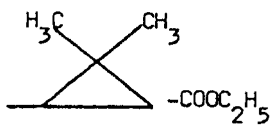 | H | Br | Br | Br | 4.1 (q, 2H, $CH_2$); 3.7 (d, 1H, CH-OH); 3.0 (s, broad, 1H, OH); 1.8-1.6 (m, 2H, CH-CH); 1.4-1.2 (m, 9H, $CH_3$) | |
| 22 | 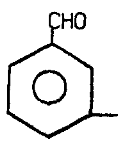 | H | Cl | Cl | Cl | 10.6 (s, 1H, CHO); 8.1-7.4 (m, 4H, Arom); 5.3 (s, 1H, CH); 4.7 (broad 1H, OH) | |
| 23 | 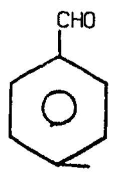 | H | Cl | Cl | $CF_3$ | 9.9 (s, 1H, CHO); 8.1-7.8 (q, 4H, Arom); 5.5 (s, 1H, CH); 3.8 (broad, 1H, OH) | |
| 24 | 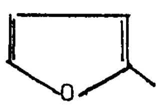 | H | Cl | Cl | $CF_3$ | 7.4 (t, 1H) + 6.4 (dd, 2H, Arom); 5.2 (d broad, 1H,CH); 3.8 (d, broad, 1H, OH) | |

EXAMPLE 25

Preparation of ethyl 2-(2-chloro-3,3,3-trifluoro-2-propenyl)-3,3-dimethyl-cyclopropanecarboxylate.

1.6 g (5 millimoles) of ethyl 2-(2,2-dichloro-1-hydroxy-3,3,3-trifluoro-propyl)-3,3-dimethyl-cyclopropanecarboxylate, prepared as described in example 4, were dissolved in 10 ml of DMF and 5 ml of pyridine; 1 ml of acetic anhydride was added to the mixture, that was kept at room temperature for 2 h under stirring.
When the whole carbinol was converted into acetate (gas-chromatographic control), 520 mg (8 milligram atoms) of zinc in powder were added and one heated at 60°C for 2 h. The mixture was then treated under the usual conditions and the residue was purified in a chromatographic way on silica gel; 1.2 g of the desired product were obtained.
$^{1}$H-NMR ($CDCl_3$, TMS, $\delta$ ppm): 1.1-1.4 (9H, m); 1.65-2.55 (2H, m); 4.1 (2H, q); 5.7-6.2 (1H, m).

EXAMPLE 26

Preparation of 3,5-dichloro-4-(2-chloro-3,3,3-trifluoro-1-propenyl)-aniline (intermediate for insecticides).

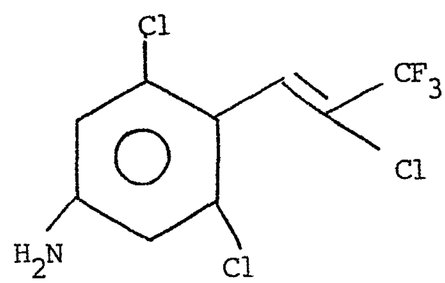

3.5 g of 2,2-dichloro-3,3,3-trifluoro-1-(2,6-dichloro-4-nitrophenyl)-1-propanol, prepared as described in example 9, were dissolved in pyridine (15 ml) and treated with 1.5 g of acetic anhydride.
After 3 h the whole was poured into water and ice and then it was extracted 3 times with ether using 70 ml at a time. The ethereal phase was washed with N/10 HCl in order to eliminate the excess of pyridine, and neutralized with sodium bicarbonate in a saturate solution, then the ether was removed thereby obtaining 3.4 g of acetate. 0.6 g of

acetate were treated with 0.95 g of iron in powder in 6 ml of ethanol and 1.5 ml of $H_2O$ acidified with 0.05 ml of concentrated HCl. The mixture was heated at 80°C for 4 h, then poured into water, brought to pH 9 and extracted 3 times with 50 ml of ethyl ether at a time. After drying on sodium sulphate 0.33 g of aniline derivate were obtained.
$^1$H-NMR ($CDCl_3$, TMS), (ppm): 3.9 (2H broad); 6.6 (2H, s); 7.2 (1H, s).

EXAMPLE 27

Preparation of 2-chloro-3,3,3-trifluoro-1-phenyl-1-propene

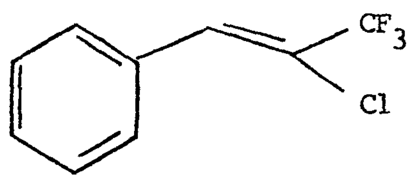

20 ml of DMF, 3500 g of benzaldehyde and 3160 g of $CF_3CCl_3$ were loaded into a 50 l flask under a nitrogen atmosphere. The temperature was brought to -5°C, 2 g of bisublimed $I_2$ was added and then in small shares, over about 2 h, Zn (3313 g) in powder form previously activated in order to restrain the reaction exothermicity.
The temperature was let reach 15°C and after 15 minutes 5 l of acetic anhydride were added to the mixture. The temperature was brought to 55°C and after 1 h a further amount of Zn in powder form (2800 g) was added. It was heated at 80° for 1 1/2 h, then cooled, and the whole was poured into $H_2O$, acidified with HCl and extracted at room temperature with 30 l of $CH_2Cl_2$ (twice).
The organic extract was washed till neutral pH and concentrated. The residue (6920 g) was distilled at reduced pressure thereby obtaining 5185 g of the product (m.p. 61°C at 4 mmHg).

CLAIMS:

1) A process for preparing polyhalogenated carbinols having the general formula:

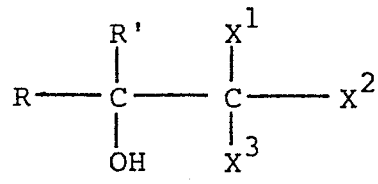

(I)

characterized in that a carbonylic compound having the formula:

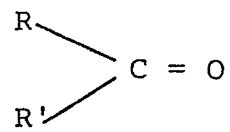

(II)

is reacted with a system consisting of a polyhalogenated hydrocarbon having the formula:

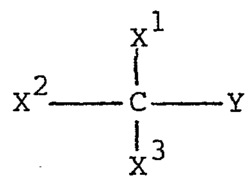

(III)

and of a divalent metal or of a metal salt, in an aprotic dipolar solvent, at temperatures ranging between -20°C and the boiling temperature of the solvent, in said formulas

- R represents: a H atom, an alkyl, cycloalkyl, alkenyl, cycloalkenyl, alkadienyl group, said groups may be substituted, preferably at the first two carbon atoms of the aliphatic chain, by one or more groups selected from a halogen atom, aryl groups, -CN, $-NO_2$, -COOR", -COR", wherein R" is H or a $C_1$-$C_4$ alkyl group, said R may further represent a phenyl, naphthyl, anthracyl group, a heterocyclic radical, each optionally substituted by one or more groups selected from halogen atoms, alkyl, alkenyl, alkoxyl, $-NO_2$, -CN, haloalkyl, haloalkenyl, haloalkoxy, thioalkyl, alkylamino, dialkylamino, -COOR", -COR" groups;
- R' represents a H atom, a haloalkyl, -CN, -COOR" group;
- $X^1$ represents Cl, Br;
- $X^2$ represents F, Cl, Br;
- $X^3$ represents Cl, Br, $CF_3$, $CCl_3$;
- Y represents Br or Cl when $X^1$, $X^2$ and $X^3$ are different from Br.

2) A process according to claim 1, wherein as metal zinc, copper, iron , magnesium is used and as metal salt stannous chloride is used.

3) A process according to one or both of the claims 1-2, wherein as solvent dimethylformamide, dimethylsulfoxide, hexamethylphosphorotriamide, N-methylpyrrolidone, sulfolane, N,N-dimethyl-imidazolidinone is used.

4) Polyhalogenated carbinols having the formula (I) according to claim 1, wherein $X^3$ represents $-CF_3$.

5) Polyhalogenated carbinols having the general formula:

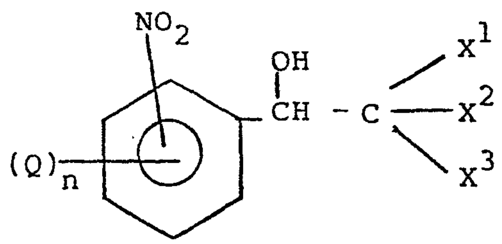

(IV)

wherein $X^1$, $X^2$ and $X^3$ have the meaning as defined in claim 1; Q represents H, a halogen atom, a $C_1$-$C_3$ alkyl group, optionally substituted by 1-3 halogen atoms, $-OCH_3$, $-SCH_3$, $-OCF_3$; n is a whole number comprised between 1 and 4.

6) Polyhalogenated carbonyls having the general formula:

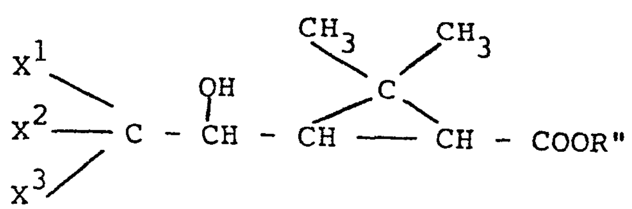

(V)

wherein $X^1$, $X^2$, $X^3$ and R" have the meaning as defined in claim 1.

7) A process according to one or more of the claims 1-3, wherein the polyhalogenated carbinols having formula (I) are successively reduced to obtain alkenes having the general formula:

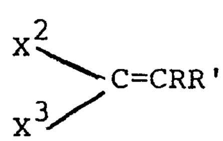

(VI)

wherein $X^2$ , $X^3$, R and R' have the meaning as defined in claim 1.